# EUROPEAN PATENT SPECIFICATION

(11) **EP 4 526 272 B1**
(45) Date of publication and mention of the grant of the patent: **29.04.2026**
(21) Application number: 23726994.9
(22) Date of filing: 15.05.2023
(51) Int. Cl.: C04B 18/02, C04B 28/02

(54) **COMPOSITION SUITABLE FOR SELF-HEALING CONCRETE, METHOD FOR MANUFACTURE THEREOF, AND USE**
ZUSAMMENSETZUNG ZUR SELBSTHEILUNG VON BETON, VERFAHREN ZU IHRER HERSTELLUNG UND IHRE VERWENDUNG
COMPOSITION APPROPRIÉE POUR BÉTON AUTORÉPARABLE, SON PROCÉDÉ DE FABRICATION ET UTILISATION

(30) Priority: 16.05.2022 EP 22173460
(43) Date of publication of application: 26.03.2025
(73) Proprietor: Purac Biochem B.V., 4206 AC Gorinchem (NL)
(72) Inventor: DE VOS, Siebe Cornelis, 4206 AC GORINCHEM (NL); KOK, Symone, 4206 AC GORINCHEM (NL)
(74) Representative: De Vries & Metman
(86) International application number: PCT/EP2023/063000
(87) International publication number: WO 2023/222616

(56) References cited:
- WO-A1-2011/126361
- WO-A1-2016/010434
- US-A1- 2014 248 681
- US-A1- 2015 122 486

## Description

The invention relates a particulate composition suitable for self-healing concrete applications, also indicated herein as an agent for self-healing concrete. The invention also relates to a method for manufacturing such composition, and to the use thereof in virgin concrete and concrete repair.

Concrete is a strong and sustainable material, which currently is widely used, for example in art, house, office, road and other civil constructions, like bridges, cellars and parking garages. Generally, concrete is composed of a cured mixture of cement with additives such as sand, water and (optionally) gravel. The material has a high compressive strength and a rather low tensile strength, provides a large freedom of structure formation, and it is relatively inexpensive. However, concrete has the disadvantage that it suffers from formations of tiny cracks, which cracks can cause severe affect the strength properties of concrete, and therewith the safety and the sustainability of objects made from the concrete. Especially in iron-reinforced concrete structures, water transported through such can cause oxidation of the iron reinforcement structures. The resulting expansion around the iron structures can lead to crumbling of the concrete surrounding the iron. Therefore this process can eventually lead to destruction of the concrete structure.

Because of these detrimental phenomena, concrete repair mechanisms have been extensively studied and repair processes have been developed. These repair processes can be preventive (to be applied before crack formation occurs) or curative (to be applied after crack formation). Of the many approaches described in literature, an interesting and promising method pertains to the use of special bacteria or (dormant) spores of such bacteria. When these bacteria are supplied with certain substrates, they appear to be able to produce lime stone or other stone types under the conditions of crack formation in the presence of nutrients. When applied in and/or on concrete, mixtures of bacteria (spores), nutrients (for bacteria growth) and substrates (for the production of lime stone), appear to be able to prevent cracks, or to repair (or heal) such cracks after their formation. In case of crack formation, such formed cracks can be fully repaired, so that the strength of the damaged concrete material can be improved and possibly even fully restored to the original strength. Thus, as a preventive measure against future crack formation in cured concrete constructions, such mixtures (or agents) can be added to and/or mixed with virgin, uncured concrete. However, as a curative measure, such mixtures (or agents) can also be applied to already existing cracks in cured concrete.

WO2011126361-A1 describes a process for the production of a cementious material, which comprises mixing cement starting materials and a particulate healing agent. The healing agent comprises coated particles, especially formed as tablets, wherein these particles contain bacterial material and one or more additives. The bacterial material may be composed of (lyophylized) bacteria or its spores. The additive may comprise a calcium compound and optionally organic compounds, trace elements and/or phosphor compounds. The particles or tablets are coated with a cement- and contrete-compatible layer, which may comprise a (co)polymer based coating based on one or more monomer types selected from the group comprising glycolide, lactide, ε-caprolactone, δ-valerolactone, N-vinylcaprolactam, 3,6-dimethyl-1,4-dioxane-2,5-dione, glycosyloxyethyl methacrylate, 1,6-bis(p-acetoxycarbonyl-phenoxy)hexane, and (3S)-cis-3,6-dimethyl-1,4-dioxane-2,5-dione. In the example an epoxyresin coating is used. The particles are manufactured using a two-step process, comprisisng a first step of compressing the bacterial material and one or more additives to form particles or tablets, followed by application of the coating.

US2014248681 describes microcapsules, for inclusion in concrete, adapted to reduce the area of a defect by at least 45 percent in said concrete once a quantity of said microcapsules has ruptured, said microcapsules each comprising: a polymeric shell encapsulating a liquid core, wherein the polymeric shell comprises a substantially impermeable polymer layer and the liquid core comprises carbonatogenic bacterial spores, and optionally bacterial nutrients, dispersed in a liquid medium. Also disclosed is a concrete composition comprising a quantity of such microcapsules, and a method of reducing the area of a defect in concrete. The polymer is selected from gelatines, polyurethanes, polyolefins, polyamides, polysaccharides, silicone resins, epoxy resins, chitosan, and aminoplast resins.

US2017044420A describes a method for delivering a microbe or enzyme to a selected location, in particular downhole in well cementing applications, which comprises conveying a coated aggregate to a selected location; wherein the coated aggregate comprises an aggregate and a coating disposed on the aggregate; the coating comprising a polymer matrix and a calcium carbonate producing agent comprising a microbe, an enzyme. Exemplary thermoplastics cited for use in the polymer matrix include polyethylene, acrylonitrile-butadiene styrene, polystyrene, polyvinyl chloride, fluoroplastics, polysulfide, polypropylene, styrene acrylonitrile, nylon, and phenylene oxide. Exemplary thermosets include epoxy, phenolic, polyester resin, polyurethanes, epoxy-modified phenolic resin, and derivatives thereof.

The healing particles and their manufacture described in the art have a number of disadvantages. A first disadvantage resides in their manufacture, which often requires the performance of a separate coating step. A further disadvantage resides in the fact that the coating is required to crack to release the active compounds. A still further disadvantage resides in the fact that many of the cited polymers are not biodegradable.

It is an object of the invention to overcome or at least mitigate the problems associated described for known healing agents. More in particular, the invention aims at providing an agent that can suitably be used in pre-cured concrete in order to avoid or diminish crack formation in the cured or solid concrete, which can be manufactured in a simple and cost-effective manner, which does not rely on destruction of a coating, and which relies on biodegradable and renewable materials. The healing agent should also be suitable for use in the repair of cracks in solid or cured concrete. The invention is also directed to provide a method for the manufacturing of the healing agent.

In one embodiment, the present invention pertains to a particulate composition suitable for self-healing concrete comprising limestone-forming bacteria or spores of limestone-forming bacteria and a bacterial nutrient dispersed in a lactic acid based oligomer matrix.

In another embodiment, the invention pertains to a method for manufacturing a particulate composition suitable for self-healing concrete comprising the steps of mixing a lactic acid based oligomer in the liquid phase with limestone-forming bacteria or spores of limestone-forming bacteria and a bacterial nutrient, and solidifying the resulting mixture to form solid particles.

In a further embodiment, the invention pertains to the use of the particulate composition in virgin concrete, and to the use of the particulate composition in concrete repair.

Not wishing to be bound by theory it is believed that the particles of the present invention act as follows when incorporated into concrete: When a crack occurs in concrete, water enters the crack. The presence of water, in combination with the alkaline environment within the concrete, results in hydrolysis of the OLA, and release of the bacteria or spores and nutrients. The bacteria grow on the nutrient and the lactate released from the OLA, and form calcium carbonate, or limestone, which closes the cracks. When the particles of the present invention are used in a composition to heal cracks in existing concrete, the particles will be applied onto the concrete in the presence of water. Again, the alkaline conditions prevailing in the presence of concrete cause hydrolysis of the OLA.

The invention will be discussed in more detail below, as will be the problems solved by and the advantages associated with the various aspects and embodiments of the invention.

The invention makes use of a lactic acid based oligomer, also indicated herein as OLA. The OLA is a polyester oligomer consisting for at least 50 mol.% of lactic acid monomers, the balance being monomers selected from lactic acid compatible monomers.

Suitable lactic acid compatible monomers may be selected from monomers having a single alcohol group and a single carboxylic acid group. Aliphatic hydroxyacids may, e.g., be used, in particular compounds 2-10, in particular 2-8, more in particular 2-6 carbon atoms. These compounds may form cyclic esters with themselves or with other compounds containing a hydroxy group and a carboxylic acid group. Accordingly, suitable monomers of this type include glycolic acid, but also glycolide, which is the cyclic ester of glycolic acid, lactones, and cyclic esters of glycolic acid and lactic acid. 2-hydroxybutyric acid is another example of a suitable compound.

Interesting further lactic acid comparable monomers may be selected from aliphatic monomers having two alcohol groups in combination with aliphatic monomers having two carboxylic acid groups.

Examples of suitable aliphatic monomers having two hydroxyl groups are alkane diols such as ethane diols, propane diols, butane diols, and glycol compounds such as ethylene glycol, diethylene glycol, and triethylene glycol. Biobased compounds, e.g., compounds produced through fermentation or derived from annually renewable resources are considered preferred.

Examples of suitable dicarboxylic acids are succinic acid, fumaric acid, adipic acid, maleic acid, oxalic acid, and malic acid.

Lactic acid compatible monomers selected from the group of glycolic acid (often in the form of glycolide, the cyclic diester of glycolic acid), and lactones, in particular epsilon-caprolactone, are considered preferred.

It is preferred for the OLA to consist for at least 70 mole% of lactic acid monomers, in particular at least 80 mole%, more in particular at least 90 mole%, in some embodiments at least 95 mole%, or at least 99 mole%.

As the lactic acid serves as the substrate for the bacteria, higher amounts of lactic acid are considered preferred.

In general, the OLA will have a number-average molecular weight of less than 50 kg/mol as determined by conventional gel permeation chromatography (GPC) relative to PS standards, measured in CHCl₃ with RI detection. If the number-average molecular weight is above that value, the viscosity of the polymer in the liquid phase will be relatively high, making it difficult to mix the OLA with the further components. The number-average molecular weight generally is at least 1 kg/mol. If the number average molecular weight is below that value, it will be difficult to manufacture suitable particles.

It is preferred that the number-averaged molecular weight (Mn, rel. PS) of the OLA is in the range of 5-30 kg/mole, in particular 10-25 kg/mol.

In the manufacturing of the particles according to the invention, the OLA will be mixed with the further components in the liquid phase, i.e. above the melting point or the glass transition temperature of the OLA. As the bacteria or bacteria spores may be sensitive to higher temperatures, especially when mixing times and processing times are longer, it is preferred for the OLA to have a relatively low processing temperature. For this reason, and for further reasons discussed below, it is preferred for the OLA to be amorphous, as amorphous OLAs have a glass transition temperature which is relatively low, especially when compared with the melting point of crystalline OLAs.

The melting temperature (for crystalline materials) and the glass transition temperature depend on the molecular weight of the OLA and on its chemical composition.

As indicated above, the OLA is based for at least 50 mole% on lactic acid monomers. Lactic acid can exist as two different enantiomers (L and D). The optical purity of the OLA is higher in case that it consists substantially of only one of these two enantiomers. OLAs of high optical purity have the ability to crystallize in solid phase and therefore can show relatively high melting temperatures, often even more than 140ºC. OLAs with lower optical purity often do not or hardly have the ability to crystallize and thus may have melting temperatures only between 110 and 140ºC. When the optical purity is low - for example when the L/D enantiomer ratio is in the range of 85/15 to 15/85 - crystallization is no longer possible and in the solid state the material can only exist in the amorphous state. Consequently, there is no longer a melting point and there is only a glass transition temperature - as measured by DSC - left, which, dependent on average chain length and L/D ratio, is in the range between 30-60ºC. Above 200ºC there exists a realistic risk of thermal degradation of the OLAs, which is considered as a clear disadvantage.

In one embodiment, the OLA in solid form has an amorphous - *i.e.,* non-crystalline - structure. In addition of having a glass transition point and thus allowing processing at lower temperatures than the melting point, a further clear advantage of this type of OLAs is that the degradation rate of amorphous materials in concrete - and not only in concrete, but under all degradation or compositing conditions for poly lactic acid (PLA) - appears to be significantly higher than for (semi-)crystalline materials. As prepared amorphous OLAs are defined to have a net enthalpy value of no more than 5 Joule per gram (J/g) as measured by differential scanning calorimetry (DSC) in a heating run from room temperature to 200ºC at a typical rate of 5K/min. In this respect, it has appeared that it is advantageous to use OLAs which are characterized in that the OLA is a copolymer of L-lactic acid and D-lactic acid monomers, wherein the amount of the minor lactate units is at least 10% of all lactate units. OLAs answering this prerequisite do have an amorphous structure in the solid state. Preferably the amount of the minor lactate units range between 10% and 30% of all lactate units in the OLA.

In one embodiment, the OLA is a product obtained from depolymerised PLA material. For this purpose recycled PLA material or off-spec PLA material can be used, which is depolymerised from high molecular PLA materials to OLA material having the required molecular weight. Using such material reduces material costs.

The self-healing agent of the present invention comprises limestone-forming bacteria or spores of limestone-forming bacteria and a bacterial nutrient dispersed in a lactic acid based oligomer matrix. Obviously, bacterial and spores may also be used in combination (even if not explicitly stated elsewhere in this specification. The use of spores may be preferred, as they generally are less temperature-sensitive than bacteria.

The specific type of limestone-forming bacteria or spores of limestone-forming bacteria is not critical to the present invention. Particularly suitable organisms combine spore formation capabilities, with the spores being resistant to high temperatures and elevated pH values, and a capability to ferment lactic acid under aerobic or facultative anaerobic conditions.

Bacteria used in the state of the art, e.g., as described in US2017044420 may be used in the present invention also. Examples of suitable organisms include those from the genera such as *Bacillus* sp. and *Sporosarcina* sp. Specific examples include *Sporosarcina pasteurii* (formerly known as *Bacillus pasteurii*)*, Bacillus megaterium, Bacillus sphaericus, Bacillus subtilis. Sporosarcina pasteurii* may be particularly preferred.

The bacterial nutrient used in the present invention generally comprises nutrients for bacterial growth. Suitable nutrient compositions are known in the art.

An interesting embodiment of the present invention is characterized in that the bacterial nutrient comprises yeast. This organic material has proven to be very useful in agents for self-healing concrete applications. The yeast has an important role in the growth and multiplication of the bacterial spores and/or bacteria. The use of yeast has appeared to be superior to the use of comparative bacterial nutrients, such as urea.

The particulate composition of the present invention generally has a particle size distribution which is such that at least 90 wt.%, in particular at least 95 wt.% of the particles has a diameter between 5.0 mm and 0.50 mm, in particular between 3.0 mm and 0.50 mm, more in particular between 2.0 and 0.50 mm. The particle size distribution is determined via sieve analysis. The particle size of the composition according to the invention can be relatively narrow, with a limited number of fines and very large particles. This results in a composition with attractive properties, e.g., good mixing behaviour when it is mixed into a concrete composition, and no dusting. It is within the scope of the skilled person to select a suitable particle size, depending on the properties of the concrete into which the composition is to be mixed.

Preferably, the particulate composition of the present invention comprises at least 95 wt.%, preferably at least 97 wt.% of OLA, at least 1.0 and at most 3.0 wt.% nutrient and at least 0.01 and less than 0.5 wt.% of bacteria or bacterial spores. Agents comprising at least these three components in the indicated amounts have shown to demonstrate excellent results in concrete repair experiments.

It is a particular feature of the composition of the invention that the bacteria or spores and the nutrients are uniformly mixed with and embedded in an OLA matrix. The particles have a homogeneous composition, and do not show the core-shell structure as can be seen in prior art particles. This means that the release profile of the bacteria or bacteria spores, the nutrient, and the OLA degradation product is relatively homogeneous over time.

The obtained agent material contains numerous bacteria or spores and numerous nutrient grains per particle. This is different from a composition in which spores and/or nutrients are individually encapsulated in a coating, resulting in a composition comprising microparticles containing nutrients or bacteria or spores thereof.

In another embodiment, the invention pertains to a method for manufacturing a particulate composition suitable for self-healing concrete comprising the steps of mixing a lactic acid based oligomer (OLA) in the liquid phase with limestone-forming bacteria or spores of limestone-forming bacteria and a bacterial nutrient, solidifying the resulting mixture, and forming solid particles.

The bacteria or bacterial spores and the nutrient can be added separately to the OLA in liquid phase or as a mixture. In case of separate additions, there is no preference for the sequence of adding these components. In one embodiment, the nutrients and bacteria or spores are combined to form a premix, and the premix is incorporated into the OLA in the liquid phase.

The nutrients and limestone-forming bacteria or spores of limestone-forming bacteria are mixed through the OLA in the liquid phase. Care should be taken that the compounds are added in a manner and at a temperature at which they do not degrade. In particular, prolonged exposure of the limestone-forming bacteria or spores of limestone-forming bacteria to elevated temperature is to be avoided. Depending on the molecular weight of the OLA the liquid OLA will have a lower of higher viscosity at suitable temperatures. It is within the scope of the killed person to select suitable mixing apparatus taking the prevailing viscosity into account.

The liquid mixture comprising OLA is solidified, and solid particles are formed. In general, but not necessarily, the steps of particle formation and solidification are combined in that particles are formed when the mixture has a high viscosity, but is not completely solid yet. Suitable methods include those adapted from providing particles of polymer compositions. It is within the scope of the skilled person to select a suitable method.

The method according to the present invention is believed to be relatively cost-effective compared with known methods as no individual particles or tablets need to be coated. Moreover, the solid particles prepared with the method of the present invention can be relatively small, so that they can suitably be used for the repair of tiny cracks which are formed in cured or solid concrete. Generally, it appears to be quite difficult to prepare small coated particles. Further, the inclusion of the bacteria and/or spores as well as the nutrient in the OLA prevents de-mixing or segregation of these components when the agent is stored in large amounts for long times. Such de-mixing or segregation can occur especially in physical mixtures of the separate components (powders) of the self-healing agent.

In one embodiment, an extruder device is used for mixing the lactic acid based oligomer in the liquid phase with limestone-forming bacteria or spores of limestone-forming bacteria and a bacterial nutrient. The use of an extruder device has the advantage that the temperature in the mixing process of the OLA, the bacterial spores and the nutrient can be carefully chosen so as to prevent damage to any of the components, and especially of the spores, during the mixing process. More in particular, an extruder device, and especially a twin-screw extruder device, has as a specific advantage that it is able to mix the mentioned ingredients at such low temperature that all ingredients, in particular the bacteria and/or bacterial spores, can survive the manufacture of the agent and can be homogenized in spite of the relatively high melt viscosity of the mixture.

In particular, the OLA can be first fed to the extruder and brought in a first part of the extruder device to a temperature where it softens and becomes in the liquid or molten phase, which temperature is usually well above the temperature at which the bacteria or bacterial spores are destructed. After the OLA has been heated in the extruder device so that it has become liquid or in molten phase, the OLA can be transported to a next part of the extruder device which has a lower temperature at which temperature the OLA is still plasticized and in a viscous liquid phase and at which temperature the bacterial spores can survive. At this part of the extruder device, the spores can be fed to the extruder and mixed with the OLA in viscous liquid or molten phase. Dependent of its constitution, the nutrient can be added before, during or after adding the bacteria or spores to the OLA. After all components have been added to the extruder device and mixed, the mixture exits the extruder, becomes solid at a lower temperature and is subsequently processed to particles.

For efficiency reasons, the OLA can be pre-heated and melted before it is entered into the extruder by melt-feeding.

In one embodiment, the bacteria or spores and the nutrient are added to and mixed with the OLA in the extruder when the temperature of the OLA in the liquid phase is in the range of 100 - 200 °C, preferably in the range between 110 and 140 °C. As discussed above, the temperature at which the OLA melts and thus reaches the liquid phase is dependent amongst others of the molecular weight of the OLA and its optical purity.

As indicated above, in one embodiment, the OLA is a product obtained from depolymerised PLA material. For this purpose, recycled PLA material or off-spec PLA material can be used, which is depolymerised from high molecular PLA materials to OLA material having the required molecular weight. Depolymerisation can be carried out using methods known in the art, e.g., using glycolysis or hydrolysis, with hydrolysis, especially under alkaline conditions, being particularly preferred. Depolymerisation conditions are known in the art and require no elucidation here.

An attractive embodiment in this context is a method in which in depolymerization and agent manufacture are carried out in a single extruder or in two connected extruders. In this case, PLA will be provided to a first part of an extruder where it is subjected to depolymerization conditions resulting in the formation of OLA in the liquid phase, followed by the provision of bacteria or spores and nutrients in a second part of the extruder or in the second extruder to form a mixture containing these components, followed by solidifying the resulting mixture, and forming solid particles.

The extruded mass can be converted to particles through methods known in the art. In one embodiment, the mass is extruded through a die plate, which is followed by a knife which chops the particles into extrudates of the desired size. If so desired, the particles can be subjected to a spheronizer treatment.

A preferred embodiment of the method according to the invention is characterized in that the solidified material is processed into solid particles by means of an underwater pelletizer. The use of such pelletizer type provides a simple and convenient manner of obtaining the solid or almost solid mixture of OLAs, bacteria or spores and nutrients as small particles. In practice, the mixed material exits the extruders in liquid or viscous form through the small bores of a hot die plate attached to the extruder, commonly preceded upstream by a gear pump for flow control and pressure generation, and by a melt filtration system or screen changer to protect the backside of the die plate from becoming blocked with impurities. The stands leaving the bores in the hot die plate solidity upon contact with the water of the pelletizer, and the solidified strands are cut into particles in the water, by means of a rotating, cutting knife which is part of the pelletizer. The particles formed are moved away from the vicinity of the die plate by means of a water flow, pass a water/granulate separation system, and are subsequently obtained as small particles that can be dried when needed. Depending on the type of pelletizer and machine details like cutter speed and mass flow through each bore, the mean particle size of the solid particles can be adjusted to less than 3 mm, preferably less than 2 mm and most preferably less than 1 mm. Underwater pelletizers are routinely applied in industrial polymer manufacturing, e.g., for polyolefins and polyesters, and can be commercially obtained from companies like MAAG Gala, BKG and Econ. The production of particles as small as 1-2 mm is generally referred to as microgranulation or micropelletisation.

The invention also relates to the use of the agents of the present invention in virgin concrete (*i.e.,* pre-cured concrete, which has not yet been hardened) as well as in concrete repair, e.g., the repair of shaped bodies of cured or hardened concrete, in which cracks have been formed.

In one embodiment, the particulate composition is incorporated into a concrete composition before shaping thereof.

In another embodiment, the particulate composition is provided onto concrete in which cracks have formed.

As will be evident to the skilled person, different embodiments of the present invention can be combined unless they are mutually exclusive. All percentages used herein are weight percentages, unless specified otherwise. When amounts, concentrations, dimensions and other parameters are expressed in the form of a range, a preferable range, an upper limit value, a lower limit value or preferable upper and limit values, it should be understood that any ranges obtainable by combining any upper limit or preferable value with any lower limit or preferable value are also specifically disclosed, irrespective of whether the obtained ranges are clearly mentioned in the context.

In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

The following examples will illustrate the practice of the present invention in some of the preferred embodiments. Other embodiments within the scope of the claims will be apparent to one skilled in the art.

### Examples

### Example 1: General preparation method

The following examples 1a) and 1b) illustrate an embodiment of the invention in which an OLA is melt-mixed OLA with 2% yeast masterbatch using an extruder device, followed by in-line shaping into micropellets (1-3 mm) by means of a lab-scale underwater pelletizer.

The OLA was a lactic acid oligomer based on > 99% lactic acid monomers, produced via ring-opening polymerization of L- and D-lactide (90/10) in batch in the presence of SnOct₂ polymerization catalyst and an initiator for molecular weight control, and provided in the form of a powder (ground lactic acid oligomer). Conventional GPC was carried out for determining the Mn and Mw, with CHCl₃ as the eluent and PS standards for calibration. The OLA had Mn 20kg/mol and Mw 30kg/mol and a glass transition temperature of 35-45ºC.

The dry powder masterbatch contained bacterial spores and yeast. This "Base+" formulation is a fine, brown powder formulation consisting of spores of CaCO₃ producing bacteria and yeast used for self-healing concrete applications. The spores remain dormant in dry state, protecting themselves against temperature/pH shifts, until they come in contact with moist (and nutrients). The spores are relatively resistant to high temperature up to 120°C for short periods of time, thus allowing for low temperature processing and/or mixing.

The extruder device was a state-of-the-art KraussMaffei Berstorff ZE 42x40D BluePower corotating twin-screw extruder (TSE). The device had a screw diameter of 42mm and a (temperature-controlled) barrel length of 40D.

The water-cooled feed port was used to add the OLA powder from a gravimetric Brabender solids feeder. After heating and plastification of the amorphous oligomer in the initial zone of the TSE, the yeast masterbatch (MB) was added using a twin-screw side feeder (type ZSE 42A) at L=20D. The solid MB was metered into the side-feeder (at 75rpm) using a Colortronic twin-screw feeding device. Further downstream, melt-mixing for gentle homogenization of the OLA and the yeast MB was achieved using a conveying screw configuration equipped with two mild melt sections. The first consisted of a neutral kneading element (type 42BP-KB1.25/5/45L/2), followed by a narrow, reversed mixing element (type 42BP-KB0.75/5/45R/2). The second section consisted of an identical neutral kneading element (type 42BP-KB1.25/5/45L/2), followed by a positive mixing element (type 42BP-KB0.75/5/45L/2) and again a reversed mixing element (type 42BP-KB0.75/5/45R/2 ).

At the die of the extruder, there was a breaker plate with coarse (400 micron) filter mesh, a gear pump, and a diverter valve in front of the underwater pelletizer (Gala LPU) used to make microgranulate (micropellets).

Typically, the throughput rate of the extruder was set at 15-30kg/hr for the OLA powder, while the screw rotation speed was set at 100-125rpm. The addition level of the yeast MB was 2%, so 0.6kg/hr at a feeding rate of 30kg/hr.

The extruder barrel temperature profile was, starting from the water-cooled feed zone: 25-70-125-120-110-110-110-120 (all in ºC), while the adapter, filter, melt pump, diverter valve and die head of the LPU UWP were set at 125-120-120-120-200 (all in ºC), respectively.

Further key data of the Maag-Gala LPU underwater pelletizer were as follows: the die plate had 12 bores with a diameter of 0.8mm, the 7-spoke cutter hub was set at 5000rpm and the cooling water temperature was controlled at 20-40 ºC.

### Example 1a

Microgranulate of the OLA described above, first without addition of the 2% yeast and spores MB, was produced using the described set-up running at 15kg/hr and 100rpm is follows. The die plate temperature of the pelletizer was 230°C and the cooling water was at 30°C. With low internal pressures (10 bars) before the die plate and filter screen and an OLA melt temperature of 135°C, OLA microgranulate was produced in a stable operation. A particle size distribution of the solid, spherical microgranulate particles was determined by passing known amount of the product over a stack of sieves with different mesh sizes using a standard, laboratory sieve shaker (Haver & Boecker). The data from these mechanical sieving fractions depicted in Table 1 for Ex. 1, show that >98 wt.% of the microgranulate had a particle size between 1.25 and 0.71 millimeter. For comparison, OLA powder after mechanical grinding typically has an average particle size of 500 micron with all 95 wt.% <850micron. So the microgranulation process allows production of OLA particles with fewer fines and a much larger fraction of particles in the preferred size range of a few millimeters.

### Example 1b

Microgranulate of OLA mixed with 2% of yeast masterbatch (375 gr/hr) was produced using the described extruder set-up running at 15kg/hr and 100rpm as follows. The die plate temperature of the pelletizer was 275°C and the cooling water temperature was 33°C. With low internal pressures (10 bars) before the die plate and filter screen and a melt temperature of 135°C, microgranulate was produced. The microgranulate was slightly darker than the pure OLA indicating the presence of the yeast and spores.

The particle size data from these hand sieving fractions shown in Table 1 for Ex. 1b, show that about 36 wt.% of the microgranulate was larger than 1.25mm, some 95 wt.% was larger than 1 millimeter, and 64 wt.% of the microgranulate had a particle size between 1.25 and 0.710 millimeter.

**Table 1: Typical particle sizes of OLA and OLA/MB 98/2 microgranulate**

| **Sieve size (micron)** | **>1250** | **>1000** | **>710** | **Fines** |
|---|---|---|---|---|
| **Sample** | wt.% | wt.% | wt.% | wt.% |
| Ex. 1a 100% OLA | 1.7 | 64 | 34 | 0.4 |
| Ex. 1b 98/2 OLA/MB | 35.8 | 60.9 | 3.5 | 0.8 |

### Example 2

This example illustrates an embodiment of the invention at an increased scale compared to Example 1.

An 8-barrel compounding extruder, a KraussMaffei Berstorff ZE 42x36D BluePower corotating twin-screw extruder (TSE) was used, having a screw diameter of 42mm and a (temperature-controlled) barrel length of 36D.

The extruder had a polymer feed location in the main throat (barrel 1 in the solids conveying section) and an additive feed location in barrel 5. Solid yeast masterbatch (MB) was added in barrel 5. Venting was done in barrel 4, to facilitate the backward removal of residual moisture and air from the yeast MB. The vacuum pump connection for degassing the extruder was located in barrel 7.

A single-screw, gravimetric Brabender solids feeder was used to add the OLA powder at the polymer feed location. After heating and softening of the amorphous oligomer in the initial zone of the TSE, the yeast masterbatch (MB) was added using a top feeder (Brabender loss-in-weight feeder) with a single screw and agitator. Further downstream, melt-mixing for gentle homogenization of the OLA and the yeast MB was achieved using a conveying screw configuration equipped with two gentle melt-mixing sections to achieve proper distributive mixing of the yeast additive in the OLA melt.

The powder feeding zone of the extruder secured stable powder feed into the melting zone section. Besides positive and reversed mixing elements, a pressure build-up zone at the end of the extruder was used to secure high enough inlet pressure before the gear pump .

At the die of the extruder, there was a screen changer with 80 mesh filter, a gear pump, and a diverter valve in front of the underwater pelletizer (UWP, type MAAG SPHERO^{®}100) used to make solid microgranulate (micropellets).

Typically, the mass throughput rate of the extruder was set between 95 - 415 kg/hr for the OLA powder, while the screw rotation speed was set between 200 - 900rpm. The addition level of the yeast MB was typically set at 2.1%. For example, if the total throughput rate of the extruder was 180 kg/hr, the yeast MB was fed at a rate of 3.8 kg/hr and the OLA powder at 176.2 kg/hr, respectively, to arrive at a 2.1/97.9 mass ratio.

The extruder barrel temperature profile was, starting from the water-cooled feed zone: 30-100-120-120-120-140 (all in degrees Celsius), while the downstream adapter, filter, melt pump, diverter valve and die head of the UWP were set at 125-120-120-120-210 (all in degrees C), respectively.

The UWP was a MAAG SPHERO^{®}100 fitted with 12 cutting blades while the die plate always had 192 holes. The two selected die plate holes had a diameter D of (0.65mm and 0.5mm),1 2-spoke cutter hub, and it was operated with a rotating speed of 3500 rpm - 4800 rpm and the cooling water temperature was controlled at 20 - 40 degrees Celsius. The rotating speed of the cutter hub of the UWP was adapted to the process conditions, like total mass throughput rate, and used to control the average particle weight of the granulate in the target window.

### Example 2a

Microgranulate of pure OLA was produced using the described extruder set-up running at 180kg/hr and 380rpm as follows. The selected die plate size had 192 holes of 0.65 mm diameter, temperature of the die plate was set at 210 °C and the cooling water temperature was kept at 30°C. With low internal pressure (<10 bar) before the die plate and filter screen (80 mesh) and with a melt temperature of 140°C, solid microgranulate was produced.

### Example 2b

Microgranulate of OLA mixed with 2.1% of yeast masterbatch (MB) was produced using the described extruder and processing parameters as described in Ex. 2a. The gravimetric top feeder for the yeast MB was set at a feeding rate of 3.78 kg/hr and for the OLA powder the feeding rate was set at 176.22 kg/hr. The microgranulate was slightly darker than the pure OLA after melt extrusion, indicating the presence of the yeast and spores.

### Example 2c

Microgranulate material of OLA mixed with yeast masterbatch (MB) was produced using the extruder set-up and processing conditions as described in Ex. 2b, except for the loading of yeast and spores MB, which was target to 4 %, resulting in a yeast MB feeding rate of 7.2 kg/hr and 172.8 kg/hr for the OLA powder.

### Example 2d

Microgranulate of OLA mixed with 2.1% of yeast masterbatch from this example was produced using the same extruder conditions described in Ex. 2b, except that the selected die plate holes diameter of the UWP was 0.5 mm.

### Example 2e

Microgranulate of OLA mixed with 2.1% of yeast masterbatch from this example was produced using the same extruder set-up and UWP from Ex. 2a, except that the extruder throughput rate was set at 425kg/hr and screw speed of 900rpm.

The particle size data shown in Table 2 were measured using a Malvern particle size analyser. The microgranulates of OLA with MB produced by extrusion and underwater granulation show comparable particle size distributions, and as desired, with the 90% below 1600 microns and more than 700 microns.

The results in Table 2 also show comparable molecular weight data (Mn, Mw and molecular weight distribution) of all the microgranulates samples as determined by GPC analysis, showing that the increase in throughput rate and the screw speed of the extruder does not change the molecular weight values when comparing the molecular weight values with the pure, unprocessed OLA C10 powder.

**Table 2: Malvern particle size data and GPC data of OLA and OLA/MB microgranulate**

| **Sample name** | **Die plate hole D** | **Screw speed** | **Throughput rate** | **MB (w/w)** | **Dx (10)** | **Dx (50)** | **Dx (90)** | **Mn^{a}** | **Mw^{b}** | **PDI^{c}** |
|---|---|---|---|---|---|---|---|---|---|---|
| | **(mm)** | **(rpm)** | **(kg/hr)** | **(%)** | **(µm)** | **(µm)** | **(µm)** | **(kg/ mol)** | **(kg/ mol)** | **(-)** |
| OLA C1 0 powder | - | - | - | - | 142 | 565 | 1700 | 16.2 | 26.3 | 1.62 |
| Ex. 2a) 100 OLA | 0.65 | 380 | 180 | 0 | 886 | 1120 | 1430 | 16 | 26 | 1.63 |
| Ex. 2b) 97.9/2.1 OLA/MB | 0.65 | 380 | 180 | 2.1 | 930 | 1150 | 1450 | 16.1 | 26.1 | 1.62 |
| Ex. 2c) 96/4.0 OLA/MB | 0.65 | 380 | 180 | 4 | 889 | 1140 | 1500 | 16.2 | 26.1 | 1.61 |
| Ex. 2d) 97.9/2.1 OLA/MB | 0.50 | 380 | 180 | 2.1 | 730 | 971 | 1340 | 15.8 | 25.9 | 1.64 |
| Ex. 2e) 97.9/2.1 OLA/MB | 0.65 | 900 | 425 | 2.1 | 933 | 1200 | 1580 | 16.1 | 26.1 | 1.62 |
| ^{a}Mn: number average molecular weight determined by GPC against PS standards, CHCl3 solvent. | | | | | | | | | | |
| ^{b}Mw: weight average molecular weight determined by GPC against PS standards, CHCl3 solvent. | | | | | | | | | | |
| ^{c}PDI: Polydispersity, determined by the ratio of Mw/Mn. | | | | | | | | | | |

## Claims

1. Particulate composition suitable for self-healing concrete comprising limestone-forming bacteria or spores of limestone-forming bacteria and a bacterial nutrient dispersed in a lactic acid based oligomer matrix.

2. Composition according to claim 1, wherein the bacterial nutrient comprises yeast.

3. Composition according to any one of the preceding claims, wherein the lactic acid based oligomer (OLA) has a number-average molecular weight of less than 50 kg/mol as determined by GPC relative to PS standards, measured in CHCl₃ with RI detection.

4. Composition according to any one of the preceding claims, wherein the OLA in solid form has an amorphous structure.

5. Composition according to any one of the preceding claims, wherein the OLA is a copolymer of L-lactic acid and D-lactic acid monomers, wherein the amount of the minor lactate units is at least 10% of all lactate units.

6. Composition according to any one of the preceding claims, wherein the OLA has been obtained from depolymerised polylactic acid (PLA) material.

7. Composition according to any one of the preceding claims, wherein the depolymerisation of the PLA has been effected through hydrolysis.

8. Composition according to any one of the preceding claims, wherein the composition has an particle size distribution which is such that at least 90 wt.%, in particular at least 95 wt.% of the particles has a diameter between 5.0 mm and 0.50 mm, in particular between 3.0 mm and 0.50 mm, more in particular between 2.0 and 0.5 mm, the particle size distribution being determined via sieve analysis.

9. Composition to any one of the preceding claims, wherein the particulate composition comprises at least 95 wt.%, preferably at least 97 wt.% of OLA, at least 1.0 and at most 3.0 wt.% nutrient and at least 0.01 and less than 0.5 wt.% of bacteria or bacterial spores.

10. Method for manufacturing a particulate composition suitable for self-healing concrete according to any one of the preceding claims, comprising the steps of mixing a lactic acid based oligomer in the liquid phase with limestone-forming bacteria or spores of limestone-forming bacteria and a bacterial nutrient, and solidifying the resulting mixture to form solid particles.

11. Method according to claim 10, wherein an extruder device is used for mixing the OLA, the bacteria or bacterial spores, and the nutrient.

12. Method according to claim 11, wherein the bacteria or bacterial spores are added to and mixed with the OLA in the extruder device when the temperature of the OLA in the liquid phase is in the range of 100 - 200 °C, preferably in the range between 110 and 140 °C.

13. Method according to any one of claims 10-12, wherein the solidified material is processed into solid particles by means of an underwater pelletizer.

14. Use of the composition according to any of claims 1-9 in virgin concrete or in concrete repair.

## Patentansprüche

1. Zur Selbstheilung von Beton geeignete teilchenförmige Zusammensetzung, umfassend kalksteinbildende Bakterien oder Sporen von kalksteinbildenden Bakterien und einen bakteriellen Nährstoff, dispergiert in einer Oligomer-Matrix auf Milchsäurebasis.

2. Zusammensetzung nach Anspruch 1, wobei der bakterielle Nährstoff Hefe umfasst.

3. Zusammensetzung nach einem der vorhergehenden Ansprüche, wobei das Oligomer auf Milchsäurebasis (OLA) ein Zahlenmittel des Molekulargewichts von weniger als 50 kg/mol aufweist, wie durch GPC bezogen auf PS-Standards, gemessen in CHCl₃ mit RI-Detektion bestimmt.

4. Zusammensetzung nach einem der vorhergehenden Ansprüche, wobei das OLA in fester Form eine amorphe Struktur aufweist.

5. Zusammensetzung nach einem der vorhergehenden Ansprüche, wobei das OLA ein Copolymer von L-Milchsäure- und D-Milchsäure-Monomeren ist, wobei die Menge der geringeren Lactateinheiten mindestens 10% aller Lactateinheiten beträgt.

6. Zusammensetzung nach einem der vorhergehenden Ansprüche, wobei das OLA aus depolymerisiertem Polymilchsäure (PLA)-Material erhalten wurde.

7. Zusammensetzung nach einem der vorhergehenden Ansprüche, wobei die Depolymerisation der PLA durch Hydrolyse erfolgt ist.

8. Zusammensetzung nach einem der vorhergehenden Ansprüche, wobei die Zusammensetzung eine Teilchengrößenverteilung aufweist, die derart ist, dass mindestens 90 Gew.-%, insbesondere mindestens 95 Gew.-% der Teilchen einen Durchmesser zwischen 5,0 mm und 0,50 mm, insbesondere zwischen 3,0 mm und 0,50 mm, noch mehr insbesondere zwischen 2,0 und 0,5 mm aufweisen, wobei die Teilchengrößenverteilung mittels Siebanalyse bestimmt wird.

9. Zusammensetzung nach einem der vorhergehenden Ansprüche, wobei die teilchenförmige Zusammensetzung mindestens 95 Gew.-%, vorzugsweise mindestens 97 Gew.-% OLA, mindestens 1,0 und höchstens 3,0 Gew.-% Nährstoff und mindestens 0,01 und weniger als 0,5 Gew.-% Bakterien oder Bakteriensporen umfasst.

10. Verfahren zur Herstellung einer zur Selbstheilung von Beton geeigneten teilchenförmigen Zusammensetzung nach einem der vorhergehenden Ansprüche, umfassend die Schritte des Mischens eines Oligomers auf Milchsäurebasis in der flüssigen Phase mit kalksteinbildenden Bakterien oder Sporen von kalksteinbildenden Bakterien und einem bakteriellen Nährstoff und des Verfestigens des resultierenden Gemischs, um feste Teilchen zu bilden.

11. Verfahren nach Anspruch 10, wobei eine Extrudervorrichtung zum Mischen des OLA, der Bakterien oder Bakteriensporen und des Nährstoffs verwendet wird.

12. Verfahren nach Anspruch 11, wobei die Bakterien oder Bakteriensporen dem OLA in der Extrudervorrichtung zugegeben und mit diesem gemischt werden, wenn die Temperatur des OLA in der flüssigen Phase im Bereich von 100-200°C, vorzugsweise im Bereich zwischen 110 und 140°C, liegt.

13. Verfahren nach einem der Ansprüche 10-12, wobei das verfestigte Material mittels eines Unterwasser-Pelletierers zu festen Teilchen verarbeitet wird.

14. Verwendung der Zusammensetzung nach einem der Ansprüche 1-9 in Frischbeton oder bei der Betonsanierung.

## Revendications

1. Composition particulaire adaptée pour le béton auto-cicatrisant comprenant des bactéries formant du calcaire ou des spores de bactéries formant du calcaire et un nutriment bactérien dispersé dans une matrice oligomère à base d'acide lactique.

2. Composition selon la revendication 1, dans laquelle le nutriment bactérien comprend de la levure.

3. Composition selon l'une quelconque des revendications précédentes, dans laquelle l'oligomère à base d'acide lactique (OLA) a une masse moléculaire moyenne en nombre de moins de 50 kg/mol tel que déterminée par chromatographie par perméation de gel par rapport aux PS standards, mesurée dans CHCl₃ avec détection par indice de réfraction.

4. Composition selon l'une quelconque des revendications précédentes, dans laquelle l'OLA sous forme solide présente une structure amorphe.

5. Composition selon l'une quelconque des revendications précédentes, dans laquelle l'OLA est un copolymère d'acide L-lactique et de monomères d'acide D-lactique, dans laquelle la quantité des motifs lactate mineurs est d'au moins 10 % de tous les motifs lactate.

6. Composition selon l'une quelconque des revendications précédentes, dans laquelle l'OLA a été obtenu à partir d'un matériau polyacide lactique (PLA) dépolymérisé.

7. Composition selon l'une quelconque des revendications précédentes, dans laquelle la dépolymérisation du PLA a été effectuée par hydrolyse.

8. Composition selon l'une quelconque des revendications précédentes, dans laquelle la composition présente une distribution granulométrique qui est telle qu'au moins 90 % en poids, en particulier au moins 95 % en poids des particules ont un diamètre compris entre 5,0 mm et 0,50 mm, en particulier entre 3,0 mm et 0,50 mm, plus particulièrement entre 2,0 et 0,5 mm, la distribution granulométrique étant déterminée par analyse par tamisage.

9. Composition selon l'une quelconque des revendications précédentes, dans laquelle la composition particulaire comprend au moins 95 % en poids, de préférence au moins 97 % en poids d'OLA, au moins 1,0 et au plus 3,0 % en poids de nutriment et au moins 0,01 et moins de 0,5 % en poids de bactéries ou de spores bactériennes.

10. Procédé pour fabriquer une composition particulaire adaptée pour le béton auto-cicatrisant selon l'une quelconque des revendications précédentes, comprenant les étapes de mélange d'un oligomère à base d'acide lactique en phase liquide avec des bactéries formant du calcaire ou des spores de bactéries formant du calcaire et un nutriment bactérien, et de solidification du mélange résultant pour former des particules solides.

11. Procédé selon la revendication 10, dans lequel un dispositif d'extrusion est utilisé pour mélanger l'OLA, les bactéries ou les spores bactériennes, et le nutriment.

12. Procédé selon la revendication 11, dans lequel les bactéries ou les spores bactériennes sont ajoutées et mélangées à l'OLA dans le dispositif d'extrusion lorsque la température de l'OLA en phase liquide est dans la plage de 100 - 200 °C, de préférence dans la plage comprise entre 110 et 140 °C.

13. Procédé selon l'une quelconque des revendications 10-12, dans lequel le matériau solidifié est transformé en particules solides au moyen d'un granulateur immergé.

14. Utilisation de la composition selon l'une quelconque des revendications 1-9 dans du béton vierge ou dans une réparation de béton.
